# FASCICULE DE BREVET EUROPEEN

(11) **EP 0 441 952 B1**
(45) Date de publication et mention de la délivrance du brevet: **15.06.1994**
(21) Numéro de dépôt: 90914192.1
(22) Date de dépôt: 06.09.1990
(51) Int. Cl.: A61C 1/08

(54) **ENSEMBLE DE DENTISTERIE, NOTAMMENT CONTRE-ANGLE DE FRAISAGE A CONTROLE VISUEL**
ZAHNÄRZTLICHE VORRICHTUNG, INSBESONDERE BOHRER MIT VISUELLEM KONTROLLSYSTEM
DENTISTRY ASSEMBLY, PARTICULARLY FOR COUNTER-ANGLE MILLING WITH VISUAL CONTROL

(30) Priorité: 06.09.1989 FR 8911808
(43) Date de publication de la demande: 21.08.1991
(73) Titulaire: WERLY, Marc, F-75014 Paris (FR); Ringeisen, Victor, 67160 Riedseltz (FR); Bleicher, Paul, F-67500 Haguenau (FR)
(72) Inventeur: WERLY, Marc, F-75014 Paris (FR); Ringeisen, Victor, 67160 Riedseltz (FR); Bleicher, Paul, F-67500 Haguenau (FR)
(74) Mandataire: Metz, Paul
(86) Numéro de dépôt international: FR9000590
(87) Numéro de publication internationale: WO9103209

(56) Documents cités:
- EP-A- 0 326 497
- DE-A- 2 208 902
- DE-A- 3 045 162
- FR-A- 2 591 094
- US-A- 4 403 956

## Description

### Ensemble de dentisterie, notamment contre-angle de fraisage à contrôle visuel.

La présente invention se rapporte à un ensemble de dentisterie, comprenant un contre-angle de fraisage à vision directe de la zone d'intervention et contrôle visuel assisté par ordinateur.

Le fraisage dans une cavité dentaire nécessite une vision suffisante du travail effectué. Cette nécessité apparaît avec d'autant plus d'acuité que la zone d'intervention et de travail est profonde.

En raison de l'espace restreint de travail, le dentiste ne dispose pas toujours d'un champ de vision suffisamment large, clair et net pour effectuer le travail précis que réclament les préparations de fond de cavité.

Aussi, le sens tactile du praticien doit suppléer, dans la majorité des cas, au défaut d'une vision appropriée de la zone d'intervention.

Pourtant, certains endoscopes spécifiques ont été développés. Ils sont malheureusement d'un emploi peu pratique, et encombrants. De plus, il s'avère impossible de les maintenir en position angulaire constante, par rapport à l'outil de fraisage.

Par ailleurs, l'image formée ne présente pas les qualités requises pour le travail minutieux demandé.

De plus, les matériaux récents utilisés en dentisterie réclament certaines exigences de précision et de tailles si on veut les mettre en oeuvre de façon fiable et durable, telles que :
. angulation des parois
. épaisseur du matériau.

Le brevet européen n° 0 326 497 se rapporte d'abord à un manche universel renfermant une micro-caméra vidéo destinée à recevoir l'image d'une zone dentaire.

L'extrémité du manche est équipée d'une tête de vision pivotante portant un ou plusieurs objectif(s) interchangeable(s).

Seules les dernières variantes représentées sur les figures 10 à 12 montrent l'association d'une sonde à caméra vidéo avec un manche porte-outil. Plus particulièrement, la figure 12 concerne l'intégration de la caméra dans un manche porte-outil.

L'observation s'effectue sur cette variante sous une prolongation avant du manche, comme sur toutes les réalisations avec porte-outil (figures 10 à 12).

Cette solution est à déconseiller car la prolongation nécessaire au-delà de l'extrémité normale du manche constitue une gêne importante pour la mise en oeuvre de l'outil en raison de sa position dans la bouche d'un patient.

Par ailleurs, en raison même de la forme en ligne brisée du manche, il apparaît difficile, voire impossible, que l'axe optique de la caméra coïncide avec l'axe du manche. Il en découle une complication du dispositif et des difficultés constructives liées à son intégration dans le manche.

Ainsi, dans cette réalisation, on doit utiliser un objectif-tige pour former l'image sur place ou une fibre optique pour transporter l'image le long du manche.

La présente invention a pour but de remédier à ces divers inconvénients en intégrant un ensemble de surveillance optique à l'outil de fraisage rotatif utilisé par les dentistes.

A cet effet, l'invention se rapporte à un ensemble de dentisterie, notamment un contre-angle de fraisage **à** contrôle visuel de grande définition assisté par ordinateur remarquable en ce qu'il renferme dans son manche une micro-caméra vidéo recevant l'image de la zone d'intervention à travers une fenêtre optique et une surface réfléchissante renvoyant l'image dans l'axe du manche et la transmettant en mode vidéo par la fibre optique d'éclairage à un ensemble informatique et en ce que débouchent en sous-face du manche une fibre optique destinée à apporter le faisceau éclairant sur la zone d'intervention et un orifice de sortie d'air sous pression ainsi qu'un orifice de pulvérisation d'eau sous pression formant un spray dirigé vers le champ opératoire.

L'idée générale inventive consiste à intégrer dans une pièce à main de dentisterie, notamment un outil de fraisage, un dispositif de prise de vue et de restitution d'image couplé sans fibres optiques et en transmission vidéo sur une porteuse lumineuse par la fibre optique d'éclairage à un ensemble de modélisation assisté par ordinateur, permettant de superposer à l'image le contour optimum de la taille, tel que déterminé et tracé par l'ordinateur.

De nombreux avantages découlent de la présente invention, tels que ceux indiqués ci-après :
. mise en oeuvre adéquate des nouveaux matériaux synthétiques utilisés en dentisterie
. commodité d'emploi
. précision d'intervention
. utilisation possible pour le travail courant de dentisterie
. haute définition de l'image qui est transmise par signal vidéo
. suppression du faisceau de fibres optiques
. utilisation de la fibre d'éclairage pour la transmission du signal vidéo d'image.

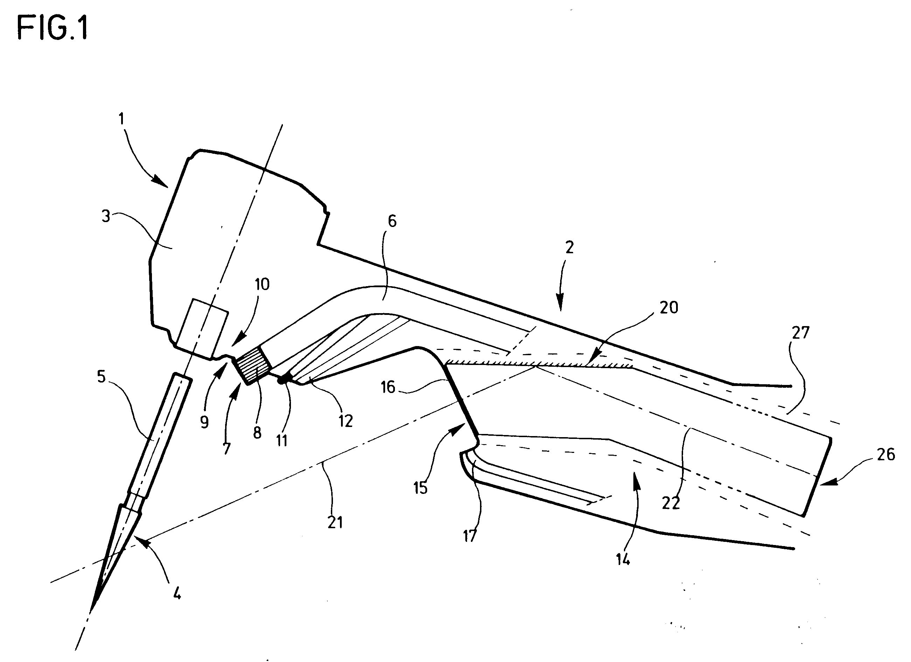

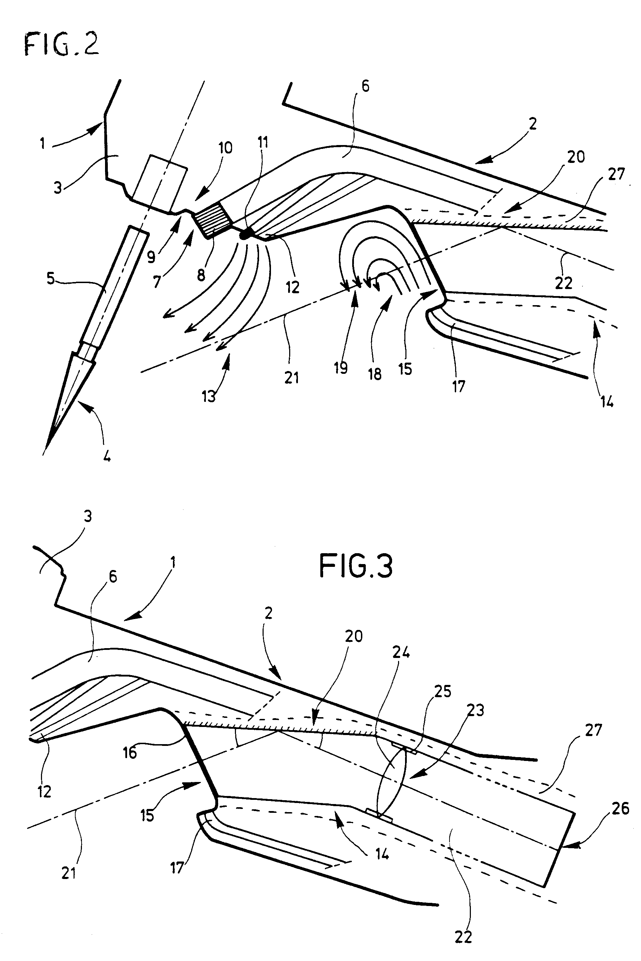

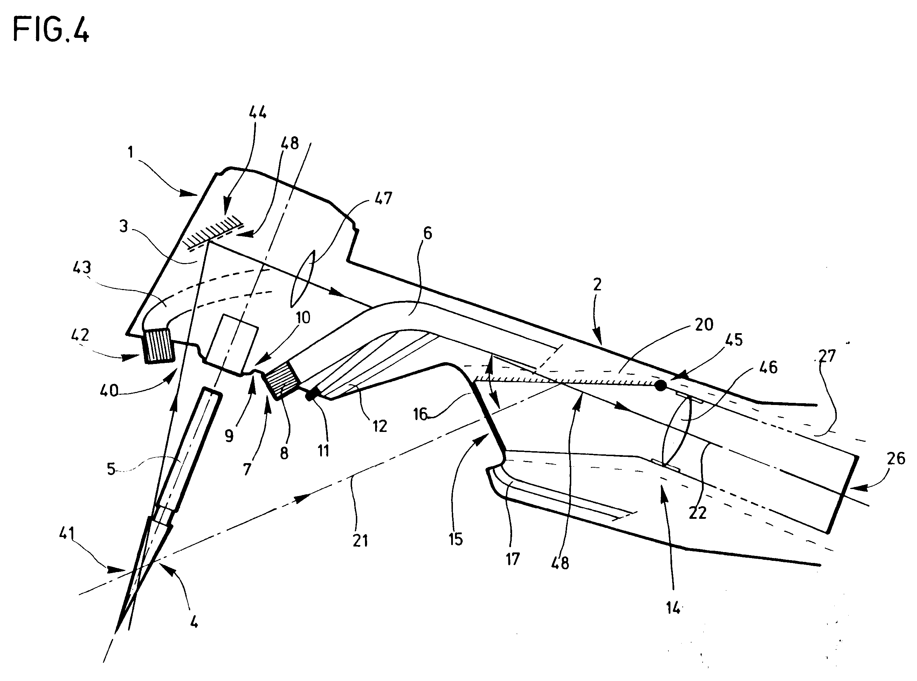

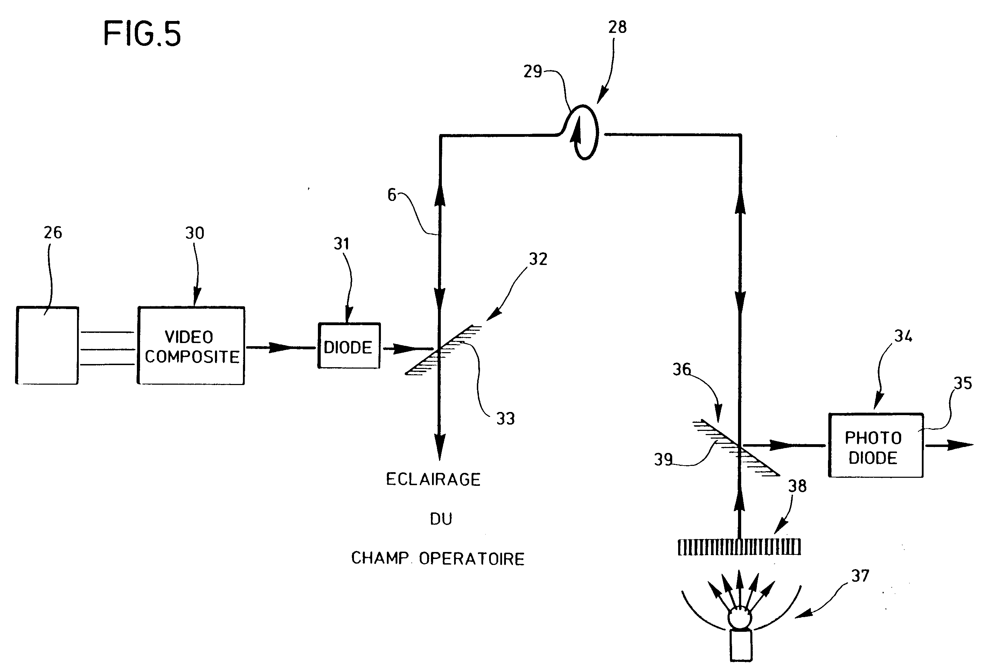

Les caractéristiques techniques et d'autres avantages de l'invention sont consignés dans la description qui suit, effectuée à titre d'exemple non limitatif sur un mode d'exécution en référence aux dessins accompagnants dans lesquels :
. la figure 1 est une vue en coupe de l'outil de fraisage à contrôle visuel ;
. la figure 2 est une coupe schématique de l'outil montrant la zone de surpression ;
. la figure 3 est une vue schématique en coupe montrant plus en détail le dispositif optique logé dans le manche ;
. la figure 4 est une vue en coupe schématique montrant une variante à double fenêtre d'observation ;
. la figure 5 est le schéma synoptique d'ensemble de la transmission du signal vidéo.

On rappellera et on développera ci-après l'idée générale inventive.

Elle consiste à intégrer dans une pièce à main de dentisterie notamment un outil de fraisage, un dispositif de vision et de prise de vues rapprochées, couplé, par la fibre optique apportant l'éclairage au champ opératoire, à un ensemble vidéo de formation et de transmission d'image, à un moniteur de restitution d'image et à un ensemble informatique comprenant un système de visualisation et un ordinateur chargé de générer et de faire apparaître en superposition sur l'écran où se forme l'image, le contour ou le tracé optimal de la taille ou du travail demandé.

Selon une autre caractéristique de l'invention, on prévoit de figer l'image sur l'écran pendant une durée déterminée, puis de la remplacer par une nouvelle image, également figée pendant la même durée, correspondant à une nouvelle vue de la zone de travail afin de supprimer le déplacement incessant de l'image sur l'écran dû aux inévitables mouvements et écarts de la main de l'opérateur.

L'image de la dent et de son contour peut alors être traitée par un programme approprié de traitement de l'image en vue de la superposer à un contour modèle ou idéal préalablement affiché à l'écran et d'obtenir par des travaux successifs de fraisage, un modelage parfait de préparation, par exemple, à la pose d'une prothèse.

Plus particulièrement, l'invention se compose des éléments et organes suivants.

Un outil 1 de dentisterie du type contre-angle, par exemple une fraiseuse, comprenant un manche 2, une tête 3 portant une fraise 4 pourvue d'une queue 5 vient s'emmancher ou s'encliqueter dans la tête 3.

Le manche 2 comporte à son intérieur une fibre optique 6 débouchant sous la tête à l'avant du manche 2 par une tête éclairante 7 réglable en orientation. Cette tête éclairante est équipée d'un filtre polariseur 8 de manière à obtenir un éclairage en lumière polarisée.

La tête éclairante 7 est disposée en biais à proximité de l'emmanchement avec l'outil sur une zone plane 9 de la sous-face 10 de l'outil.

Dans cette zone débouchent également une arrivée d'eau 11 et une arrivée d'air 12 donnant naissance à un jet pulvérisé 13.

Afin de diminuer l'effet de brouillard ou de bruine provoqué par le jet pulvérisé 13 dans la cavité de la dent en cours de fraisage, il apparaît nécessaire de placer la tête éclairante 7 entre la fraise 4 et les arrivées d'eau 11 et d'air 12. On réalise ainsi un éclairage derrière la nappe de pulvérisation-nébulisation afin de diminuer grandement les réflexions parasites, notamment celles générées par les micro-gouttes de pulvérisation. En effet, ce rideau de lumière isole optiquement le champ opératoire du milieu ambiant.

Le manche proprement dit 2 est creux. Il renferme un dispositif optique 14 de prise de vue débouchant par une fenêtre 15 inclinée par rapport à l'axe du manche et pourvue de ou constituée par un filtre analyseur 16 orienté au mieux pour bénéficier de tous les avantages de la lumière polarisée par exemple à 90°. Un conduit 17 d'air comprimé débouche à l'extrémité inférieure de la fenêtre, de façon à générer un flux d'air 18 tangentiel qui engendre une zone de surpression 19 destinée à évacuer les saletés et débris en suspension dans l'ambiance au voisinage de la fenêtre.

En raison de l'inclinaison de la fenêtre et du parallélisme, de préférence de la coïncidence de l'axe optique du dispositif optique avec l'axe du manche, un miroir supérieur 20 renvoie vers l'optique dans son axe optique l'image du champ opératoire, c'est-à-dire celle de la zone voisine de l'extrémité de l'outil.

Le degré d'inclinaison varie légèrement selon la conformation du manche et l'inclinaison entre la tête et le manche.

Si la tête 3 et le manche 2 sont perpendiculaires, l'inclinaison du miroir supérieur 20 est telle que les axes optiques incident 21 et réfléchi 22 forment avec lui un angle de 24°.

On examinera maintenant le dispositif optique.

Il s'agit d'une micro-caméra vidéo 23 permettant des prises de vue rapprochées.

Cette caméra est disposée en ligne coaxialement au manche 2. Son axe optique est parallèle et de préférence confondu avec l'axe du manche 2.

Elle comporte un objectif 24 de préférence du type zoom présentant, un servo-mécanisme 25 permettant de faire varier électriquement la focale par une action manuelle de l'utilisateur.

Le zoom 24 peut aussi bien être constitué par un objectif interchangeable par l'utilisateur à l'aide d'un mécanisme de pose et de verrouillage rapides par exemple du type à baïonnette.

La surface sensible, disposée dans le plan focal de la micro-caméra, est constituée par une matrice 26 d'éléments optoélectroniques, par exemple ceux connus sous le sigle CCD.

Le miroir supérieur 20 permet de réaliser le transfert nécessaire en orientation de l'axe optique.

On peut orienter l'axe du champ de vision en modifiant en orientation l'axe optique du miroir par un micro-mécanisme adapté commandé par un montage approprié à partir d'une action sur un dispositif électromécanique composite.

Il est important de prévoir que le volume, la forme et les dimensions de la cavité du manche servant de logement au dispositif optique soient de valeurs telles qu'aucune résonance sur une harmonique de fréquence de la turbine ou du micro-moteur d'entraînement ne soit possible.

Par ailleurs, afin de permettre la stérilisation, le dispositif optique sera convenablement protégé pour résister aux manipulations et aux agents mis en oeuvre.

A cette fin, on pourra prévoir une cavité 27 étanche à une pression d'au moins deux bars et remplie d'un gaz neutre non oxydant, par exemple d'azote.

Pour assurer le maintien d'un degré d'humidité faible, on pourra disposer dans la cavité une pastille d'un sel absorbant l'humidité.

Pour des raisons de confort et de souplesse d'utilisation le corps de l'outil présente une partie antérieure classiquement mobile par pivotement autour de la base fixe raccordée au flexible composite d'alimentation selon une articulation à pivotement 28 qui est pourvue d'un joint tournant 29 destiné à assurer la transmission des fluides et diverses informations.

Le mode de transmission du signal vidéo pourra être de deux types :
. électromagnétique par une porteuse haute fréquence
. optique par la modulation d'une diode infrarouge et transmission par la fibre optique d'éclairage pour franchir le joint tournant.

Le réseau matriciel sensible de la micro-caméra vidéo miniature du dispositif optique est exploité en formation et numérisation d'image par un montage 30 dit vidéo-composite dont le signal vidéo module une porteuse lumineuse en rayonnement, par exemple infrarouge, utilisant pour sa transmission jusqu'au dispositif optique de détection la même fibre optique 6.

Pour décrire ci-après la transmission vidéo, on se référera plus particulièrement au schéma synoptique de la transmission lumière/vidéo représenté sur la figure 5.

Une diode émettrice 31 réalise la modulation par le signal vidéo d'une porteuse lumineuse, par exemple d'une radiation infrarouge qui est injectée dans la fibre optique 6 à travers un coupleur sélectif d'émission 32.

A cet effet on peut envisager le choix d'un miroir 33 du type semi-réfléchissant, à savoir traversant pour toutes les radiations lumineuses sauf pour l'infrarouge, de manière à se retrouver dans la fibre optique 6 et à l'utiliser comme support de la porteuse jusqu'à un détecteur 34 par exemple à photo-diode infrarouge 35 qui permet de démoduler le signal vidéo avant son exploitation. Ceci après avoir convenablement isolé le rayonnement infrarouge par l'intermédiaire d'un coupleur sélectif de réception 36 disposé à proximité d'une source lumineuse distante 37 destinée à éclairer le champ opératoire après passage dans un filtre sélectif aux infrarouges 38 et son trajet le long de la fibre optique 6.

Un miroir semi-réfléchissant 39 du type de celui indiqué ci-dessus peut convenir.

Le signal vidéo alimente un moniteur vidéo pour former une image sur un écran faisant partie d'un système informatique fonctionnant sur un programme de modélisation de la taille des matériaux sur site dans le domaine dentaire en vue du contrôle visuel de la taille assistée par ordinateur.

Il est important de noter que l'on utilise comme mode de transmission du signal vidéo la modulation d'un rayon infrarouge et comme liaison de transmission du signal vidéo l'unique fibre optique 6 apportant la lumière nécessaire à l'éclairage du champ opératoire.

Afin de transmettre les signaux à travers la liaison d'articulation à pivotement 28 entre le corps de l'outil et la base fixe du manche, il y a lieu de disposer d'un joint tournant approprié équipé par exemple d'un coupleur optique périphérique ou concentrique.

Bien entendu, cette liaison peut être réalisée de façon indépendante du joint tournant sous la forme d'un raccordement distinct coaxial ou autre, flexible, semi-rigide ou autre.

A cet effet, on peut citer le raccordement d'une fibre optique dissociable du type à emmanchement, à encliquetage ou tout autre mode utilisé dans les raccords rapides.

Par ailleurs, une variante intéressante consiste à déplacer les circuits électroniques prévus dans le manche en aval du joint tournant pour les loger dans le manche en amont du joint tournant. On évite ainsi les difficultés d'alimentation et de montage et les ennuis et les parasites apportés par le joint tournant lors de la transmission du signal d'image.

Une variante de l'outil à main à vision directe dite à double observation entrant dans le même concept inventif est envisagée et représentée sur la figure 4.

Selon cette variante, on prévoit une deuxième fenêtre d'observation 40 située au-delà de la fraise 4 à l'extrémité du contre-angle 1 dont le champ de vision se situe dans une région 41 semi-cachée présentant l'ombre portée de la fraise, difficile à être vue et observée par le praticien en raison de son unique direction d'observation.

Cette deuxième fenêtre d'observation 40 possède ses propres moyens d'éclairage 42 par exemple à fibre optique 43 ou annulaire autour de la fraise et, le cas échéant, ses propres moyens optiques et de formation d'image pouvant présenter des caractéristiques optiques différentes de celles de l'optique associée à la micro-caméra 23 incorporée dans le manche 2. L'image est renvoyée grâce à un miroir avant de renvoi 44 disposé de façon à renvoyer l'image dans la direction de l'axe optique de la micro-caméra de manière que l'image renvoyée puisse être captée par la même micro-caméra et être transmise de la même façon en signal vidéo.

Le contre-angle 1 restant identique par ailleurs car il garde les moyens d'observation décrits ci-dessus, on réalise ainsi un ensemble de dentisterie à double observation de deux parties différentes du champ opératoire ou zone d'intervention d'une part dans sa partie située en deça de la fraise et d'autre part dans sa partie située au-delà de celle-ci.

Selon une version manuelle, le miroir de renvoi référencé 20 peut être réalisé mécaniquement basculant autour d'un axe 45 commandé par un bouton de manoeuvre actionné manuellement par le praticien.

Le praticien pourra ainsi voir à volonté l'une ou l'autre partie du champ opératoire située en deça ou au-delà de la fraise. Il pourra les voir plus ou moins rapprochées si l'on prévoit des dispositifs optiques séparés 46 et 47 de grossissement différents à focal fixe ou variable.

Cette version peut être améliorée en disposant à la place du miroir une lame optique 48 électro-commandée, existant dans le commerce, aux propriétés alternativement réfléchissantes et transparentes.

Ces lames optiques sont principalement réalisées à base de cristaux liquides. Elles deviennent opaques et réfléchissantes sous l'action d'un champ électrique et transparentes en l'absence du champ électrique ou inversement.

Ainsi, suffira-t-il au praticien de commander sélectivement l'application du champ électrique par un simple bouton interrupteur basculeur ou par un bouton d'application et un bouton de déconnexion du champ électrique lui permettant de passer de l'une à l'autre image de chacune des parties du champ opératoire.

On peut, bien entendu, également prévoir une telle lame optique devant le miroir avant de renvoi 44 pour envoyer ou obscurcir l'image de la partie de la zone d'intervention située au-delà de la fraise.

Le montage vidéo composite et la commande électronique de l'application du champ électrique permettent de réaliser le passage synchronisé d'une image à l'autre à un rythme élevé de succession d'images correspondant par exemple à celui du cinéma ou de la télévision.

Les images sont numérisées et transmises de la même façon en signal vidéo par modulation d'une porteuse lumineuse injectée dans la fibre optique 6 d'éclairage jusqu'à une unité de traitement dans laquelle elles sont mises en mémoire puis analysées et traitées en vue de recomposer une image unique et complète du champ opératoire sans ombre portée et, si nécessaire, sans vision de la fraise ou avec une partie de celle-ci, par exemple son extrémité.

On peut, bien entendu, également superposer les images par une vision simultanée de celles-ci.

L'avantage important de cette variante en ce qui concerne la commodité de travail du praticien apparaît immédiatement et clairement.

## Revendications

1. Ensemble de dentisterie comprenant un contre-angle de fraisage, formé d'un manche (2) terminé par une articulation de pivotement (28) et d'une tête (3) sur laquelle vient se monter un outil, par exemple une fraise (4), le manche renfermant une micro-caméra vidéo (23) destinée à recevoir dans son axe optique (22) l'image du champ opératoire provenant d'une fenêtre optique (15) éclairée par une fibre optique (6) conduisant un faisceau lumineux, l'ensemble étant couplé à un système vidéo de formation et de transmission d'image en signal vidéo démodulé et traité par une unité informatique suivie d'un moniteur de restitution d'images permettant la restitution et le traitement des images caractérisé en ce que la fenêtre optique (15) est diposée à l'extrémité du manche en deça de l'outil et renvoie directement dans l'axe du manche (2) qui coïncide avec l'axe optique (22) de la caméra (23) l'image du champ opératoire, éclairé par la fibre optique, à travers un dispositif optique (14) à surface réfléchissante, et en ce que débouchent en sous-face du manche (2) et à son extrémité avant en deça de l'outil et à côté de la fibre optique (6) prévue à l'intérieur du manche pour conduire un faisceau destiné à éclairer le champ opératoire, un orifice de sortie d'air (12) sous pression et un orifice d'eau (11) rapprochés l'un de l'autre, formant un spray dirigé vers l'extrémité de l'outil, et en ce que le signal vidéo transmettant l'image formée par la caméra vidéo (23) est constitué par la modulation d'une porteuse lumineuse qui est injectée dans la fibre optique (6) conduisant le faisceau lumineux d'éclairage du champ opératoire en vue de sa démodulation et de son traitement par un ensemble informatique suivi d'un moniteur de restitution d'images permettant la visualisation et le traitement d'images.

2. Ensemble de dentisterie selon la revendication 1, caractérisé en ce que l'extrémité de la fibre optique (15) est disposée entre l'outil porté par la tête (3) du contre-angle et les orifices (11) et (12) formant le spray.

3. Ensemble de dentisterie selon la revendication 1, caractérisé en ce que le dispositif optique (14) est une cavité coudée dans le manche, débouchant par la fenêtre optique (15) sous l'extrémité du contre-angle, comprenant un miroir supérieur de renvoi (20) convenablement incliné de façon à renvoyer l'image transmise par la fenêtre (15) dans l'axe du manche (2).

4. Ensemble de dentisterie selon la revendication 1, caractérisé en ce que la fibre optique (6) est pourvue d'un polariseur pour émettre une lumière polarisée et en ce que la fenêtre optique (15) comprend un filtre analyseur (16) permettant de s'affranchir de la polarisation parasite provenant des fines gouttelettes du spray.

5. Ensemble de dentisterie selon la revendication 1, caractérisé en ce que le signal vidéo de l'image est transporté par la fibre optique (6) apportant le faisceau d'éclairage du champ opératoire.

6. Ensemble de dentisterie selon la revendication 1, caractérisé en ce que le signal vidéo d'image module un faisceau de lumière infrarouge par une diode émettrice en lumière infrarouge, ledit faisceau modulé étant envoyé sur la fibre optique (6) par l'intermédiaire d'un coupleur optique, ledit faisceau modulé étant isolé pour être démodulé par un détecteur à photodiode en vue de son utilisation en tant que signal vidéo.

7. Ensemble de dentisterie selon la revendication 1, caractérisé en ce que, pour un contre-angle à tête perpendiculaire au manche, les axes optiques incident et réfléchi forment avec le miroir supérieur (20) un angle de 24°.

8. Ensemble de dentisterie selon la revendication 1, caractérisé en ce que la micro-caméra (23) est reliée à un moniteur vidéo en vue de la visualisation du champ opératoire.

9. Ensemble de dentisterie selon la revendication 1, caractérisé en ce que le contrôle visuel de la taille est assisté par ordinateur.

10. Ensemble de dentisterie selon la revendication 1, caractérisé en ce que le joint tournant de l'articulation de pivotement (28) est équipé d'un coupleur optique.

11. Ensemble de dentisterie selon la revendication 1, caractérisé en ce que les circuits électroniques prévus dans le manche en aval du joint tournant sont déplacés en amont du joint tournant.

12. Ensemble selon la revendication 1, caractérisé en ce que le contre-angle présente à son extrémité avant une deuxième fenêtre d'observation (40) qui est destinée à être orientée vers la partie du champ opératoire située en deça de l'outil possédant ses propres moyens d'éclairage (42), fenêtre prolongée par un miroir avant de renvoi (44) renvoyant l'image dans l'axe optique (22) de la micro-caméra (23), contre-angle sur lequel sont disposés le miroir de renvoi (20) et la micro-caméra (23).

13. Ensemble selon la revendication 12, caractérisé en ce que le miroir (20) est basculant.

14. Ensemble selon la revendication 12, caractérisé en ce que le miroir (20) est constitué par une lame optique (48) à propriétés électrocommandées réfléchissantes ou transparentes.

15. Ensemble selon les revendications 12 et 14, caractérisé en ce que le miroir avant de renvoi est recouvert d'une lame optique à propriétés électrocommandées réfléchissantes ou transparentes.

16. Ensemble selon les revendications 12, 14 et 15, caractérisé en ce que l'on fait succéder les images de l'une puis de l'autre partie du champ opératoire vues à travers l'une et l'autre fenêtre d'observation en vue de recomposer une image complète.

## Patentansprüche

1. Dentalgerät mit einem Bohrerhandstück aus einem Griffstück (2), welches an einer Drehverbindung (28) endet, und aus einem Kopf (3), auf dem ein Werkzeug befestigt wird, z.B. ein Bohrer (4), wobei das Griffstück eine Mikrovideokamera (23) enthält, die dazu dient, entlang ihrer optischen Achse das Bild des Behandlungsfeldes von einem optischen Fenster (15) zu empfangen, welches über einen ein Lichtbündel führenden Lichtwellenleiter (6) beleuchtet ist, wobei das Gerät mit einem Bildsystem zur Erzeugung und Übertragung eines Bildes in Form eines Videosignals gekoppelt ist, das mittels einer Informationseinheit demoduliert und verarbeitet wird, der ein Bildwiedergabegerät zur Wiedergabe und Verarbeitung der Bilder nachgeschaltet ist,
dadurch gekennzeichnet, daß das optische Fenster (15) an dem einen Ende des Griffstücks diesseits des Werkzeugs angeordnet ist und das Bild des durch den Lichtwellenleiter beleuchteten Behandlungsfeldes direkt in die Achse des Griffstückes (2), welche mit der optischen Achse (22) der Kamera (23) übereinstimmt, über eine optische Einrichtung (14) mit reflektierender Oberfläche zurückprojiziert, daß eine Auslaßöffnung für Druckluft (12) und eine Wasseröffnung (11), eine der anderen benachbart, unterhalb des Griffstückes (2) und am diesseitigen Ende des Werkzeugs und neben dem Lichtwellenleiter (6) münden, der im Inneren des Griffstückes zum Führen eines Lichtbündels vorgesehen ist, das zum Beleuchten des Behandlungsfeldes bestimmt ist, wobei Druckluft- und Wasseröffnung einen in Richtung des Werkzeuges gerichteten Strahl bilden, und daß das durch die Videokamera (23) gebildete Bild übertragende Videosignal durch die Modulation einer Lichtwelle gebildet wird, welche in den das Lichtbündel zum Beleuchten des Behandlungsfeldes führenden Lichtwellenleiter eingekoppelt wird, um durch eine Informationseinheit mit einem nachfolgenden Bildwiedergabegerät zur Sichtbarmachung und Verarbeitung des Bildes demoduliert und verarbeitet zu werden.

2. Dentalgerät nach Anspruch 1, dadurch gekennzeichnet, daß das Ende des Lichtwellenleiters (6) zwischen dem durch den Kopf (3) des Handstückes getragenen Werkzeug und den den Strahl bildenden Öffnungen (11, 12) angeordnet ist.

3. Dentalgerät nach Anspruch 1, dadurch gekennzeichnet, daß die optische Einrichtung (14) eine gekrümmte Höhlung in dem Griffstück ist, die in dem optischen Fenster (15) unterhalb des Handstückendes mündet und die einen passend geneigten oberen Umlenkspiegel (20) aufweist, um das über das Fenster (15) übertragene Bild in die Achse des Griffstücks (2) umzulenken.

4. Dentalgerät nach Anspruch 1, dadurch gekennzeichnet, daß der Lichtwellenleiter (6) mit einem Polarisationsfilter zum Aussenden polarisierten Lichtes versehen ist und daß das optische Fenster (15) ein Analysatorfilter (16) aufweist, um die von den feinen Tröpfchen des Strahls herrührende Störpolarisation auszufiltern.

5. Dentalgerät nach Anspruch 1, dadurch gekennzeichnet, daß das Bildsignal mittels des Lichtwellenleiters (6), welcher das Lichtbündel des Behandlungsfeldes führt, übertragen wird.

6. Dentalgerät nach Anspruch 1, dadurch gekennzeichnet, daß das Videobildsignal, das von einer Infrarotdiode stammende Infrarotlichtbündel moduliert, das modulierte Bündel durch Zwischenschaltung eines optischen Kopplers über den Lichtwellenleiter (6) transportiert und das modulierte Bündel zur Demodulation mittels eines Detektors mit Photodiode zur Verwendung als Video-Signal isoliert wird.

7. Dentalgerät nach Anspruch 1, dadurch gekennzeichnet, daß für ein Handstück mit einem Kopf senkrecht zum Griffstück die optischen Einfalls- und Ausfallsachsen mit dem oberen Spiegel (20) einen Winkel von 24° bilden.

8. Dentalgerät nach Anspruch 1, dadurch gekennzeichnet, daß die Mikrokamera (23) zum Sichtbarmachen des Behandlungsfeldes mit einem Bildmonitor verbunden ist.

9. Dentalgerät nach Anspruch 1, dadurch gekennzeichnet, daß die visuelle Kontrolle des Bohrens durch eine Datenverarbeitungsanlage unterstützt wird.

10. Dentalgerät nach Anspruch 1, dadurch gekennzeichnet, daß die Drehkupplung der Drehverbindung (28) mit einem optischen Koppler versehen ist.

11. Dentalgerät nach Anspruch 1, dadurch gekennzeichnet, daß die üblicherweise im Griffstück unterhalb der Drehkupplung vorgesehenen elektrischen Schaltungen nach oberhalb der Drehkupplung verlagert sind.

12. Gerät nach Anspruch 1, dadurch gekennzeichnet, daß das Handstück an seinem vorderen Ende ein zweites Beobachtungsfenster (40) aufweist, welches in Richtung des Behandlungsfeldteils ausgerichtet, diesseits des Werkzeugs angeordnet und mit eigenen Beleuchtungsmitteln (42) versehen ist, wobei das Fenster mittels eines vorderen Umlenkspiegels (44) verlängert ist, welcher das Bild in die optische Achse (22) der Mikrokamera (23) umlenkt, wobei der Umlenkspiegel (20) und die Mikrokamera (23) im Handstück angeordnet sind.

13. Gerät nach Anspruch 12, dadurch gekennzeichnet, daß der Spiegel (20) schwenkbar ist.

14. Gerät nach Anspruch 12, dadurch gekennzeichnet, daß der Spiegel (20) durch eine optische Lamelle (48) mit elektrisch gesteuerten Reflexions- oder Transparenzeigenschaften gebildet ist.

15. Gerät nach Anspruch 12 und 14, dadurch gekennzeichnet, daß der vordere Umlenkspiegel mit einer optischen Lamelle mit elektrisch gesteuerten Reflexions- oder Transparenzeigenschaften bedeckt ist.

16. Gerät nach einem der Ansprüche 12 bis 15, dadurch gekennzeichnet, daß die Bilder des einen und dann des anderen Teils des Behandlungsfeldes durch das eine und das andere Beobachtungsfenster zum Zusammensetzen eines Vollbildes aufeinanderfolgen.

## Claims

1. A dentistry assembly comprising a milling counter-angle formed by a handle (2) having a pivoting articulation at its end and a head (3) with a tool mounted thereon, for exemple a dental drill (4), said handle having disposed therein a micro video camera (23) for receiving along its optical axis (22) the image of the operating area through an optical observation window (15) illuminated by an optical fiber (6) transmitting a light beam, said assembly being coupled to a video system for formation and transmission of images in form of a video signal demodulated and treated through a computer unit operatively connected to an image display video monitor for the display and processing of images,
characterized in that the optical observation window (15) is situated at the end of the handle before the tool and reflects directly along the handle axis, which coincides with the optical axis (22) of the camera (23), the image of the operating area, illuminated by the optical fiber, through an optical arrangement (14) having a reflecting surface, and in that the handle (2) has a pressurized air outlet (12) and a water outlet (11) located close to one another on the underface and at the forward end of said handle before the tool and within the vicinity of the optical fiber (6) provided within the handle for transmission of a beam for illuminating the operating area, said outlets forming a spray means directed toward the tool end, and in that the video signal transmitting the image formed by the video camera (23) comprises the modulation of a carrier light beam which is injected into the optical fiber (6) transmitting the light beam for illuminating the operating area for its demodulation and processing by a computer means operatively connected to an image display monitor for image visualisation and processing.

2. Dentistry assembly according to claim 1, characterized in that the terminal end of the optical fiber (15) is interposed between the tool carried by the head (3) of the counter-angle and the outlets (11) and (12) forming the spray means.

3. Dentistry assembly according to claim 1, characterized in that the optical arrangement (14) is a cavity forming an angle within the handle, which opens out into the optical observation window (15) on the underface of the counter-angle end, comprising a superior reflecting mirror (20) adequately oriented so as to reflect the image transmitted through the optical observation window (15) along the handle axis.

4. Dentistry assembly according to claim 1, characterized in that the optical fiber (6) further comprises polarizing means for emitting polarized light and in that the optical observation window (15) comprises an analytic filter means for eliminating any parasitic polarization created by water droplets from the spray.

5. Dentistry assembly according to claim 1, characterized in that the image video signal is transmitted through the optical fiber (6) which transmits the light beam for illuminating the operating area.

6. Dentistry assembly according to claim 1, characterized in that the image video signal modulates an infrared light beam by means of an emitter diode emitting infrared light, said modulated beam being transmitted to the optical fiber (6) by an optical emission coupler, said modulated beam being isolated so as to be demodulated by a photodiode detector for subsequent use as a video signal.

7. Dentistry assembly according to claim 1, characterized in that, the counter angle being disposed perpendicular to the handle, the incident and reflected optical axis subtend an angle of 24° with respect to the superior mirror (20).

8. Dentistry assembly according to claim 1, characterized in that the micro camera (23) is operatively connected to a video monitor for visualisation of the operating area.

9. Dentistry assembly according to claim 1, characterized in that the visual size control is aided by computer means.

10. Dentistry assembly according to claim 1, characterized in that the rotatable joint of the pivoting articulation (28) is provided with an optical coupler.

11. Dentistry assembly according to claim 1, characterized in that the electronic circuits provided within the handle are located before the rotatable joint, instead of after said rotatable joint.

12. Dentistry assembly according to claim 1, characterized in that the counter-angle has a second observation window (40) located at its front end which can be oriented toward the part of the operating area which is situated beyond the tool, said second observation window being provided with its own illuminating means (42), and a front reflecting mirror (44) which reflect the image along the optical axis (22) of the micro-camera (23), the reflecting mirror (20) and the micro-camera (23) being disposed on the said counter-angle.

13. Dentistry assembly according to claim 12, characterized in that the mirror (20) is pivotably movable.

14. Dentistry assembly according to claim 12, characterized in that the mirror (20) comprises an optical foil (48) having electro-controlled optical properties which can be selectively reflective or transparent.

15. Dentistry assembly according to claim 12, characterized in that the front reflecting mirror is covered by an optical foil having electro-controlled optical properties which can be selectively reflective or transparent.

16. Dentistry assembly according to claim 12, 14 and 15, characterized in that images from one or the other of the parts of the operating area are successively transmitted through one or the other optical observation window for reconstructing a complete image.
